# EUROPEAN PATENT SPECIFICATION

(11) **EP 2 089 424 B1**
(45) Date of publication and mention of the grant of the patent: **01.07.2015**
(21) Application number: 06843366.3
(22) Date of filing: 19.12.2006
(51) Int. Cl.: C07K 14/505

(54) **METHOD FOR PRODUCTION OF HUMAN ERYTHROPOIETIN**
VERFAHREN ZUR HERSTELLUNG VON HUMANEM ERYTHROPOIETIN
PROCÉDÉ DE PRODUCTION D'ÉRYTHROPOÏÉTINE HUMAINE

(30) Priority: 06.12.2006 JP 2006329501
(43) Date of publication of application: 19.08.2009
(73) Proprietor: JCR PHARMACEUTICALS Co., LTD., Ashiya-shi, Hyogo 659-0021 (JP)
(72) Inventor: KAWASAKI, Atsuko, Kobe-shi Hyogo 655-0854 (JP); MUKAI, Keisuke, Kobe-shi Hyogo 651-1305 (JP); KIRIHARA, Sei, Miki-shi Hyogo 673-0521 (JP)
(74) Representative: Isarpatent
(86) International application number: PCT/JP2006/325983
(87) International publication number: WO 2008/068879

(56) References cited:
- EP-A- 0 267 678
- EP-A1- 1 428 878
- US-A1- 2002 146 771

### SEQUENCE LISTING

<110> JCR Pharmaceuticals Co., Ltd. KAWASAKI, Atsuko MUKAI, Keisuke KIRIHARA, Sei
<120> Method for Production of Human Erythropoietin
<130> GP98-PCT
<150> JP2006-329501
   <151> 2006-12-06
<160> 4
<170> PatentIn version 3.1
<210> 1
   <211> 28
   <212> DNA
   <213> Artificial
<220>
   <223> Nucleotides 3-8 represent an EcoRI site. Nucleotides 9-28 corres pond to the first 20 nucleotides of the coding region.
<400> 1
   ccgaattcat gggggtgcac gaatgtcc 28
<210> 2
   <211> 30
   <212> DNA
   <213> Artificial
<220>
   <223> Nucleotides 12-17 represent a BglII site. Nucleotides 18-30 corr espond to nucleotides 774-762 of the cDNA.
<400> 2
   tcaagcttct tagatctcag agttgctctc 30
<210> 3
   <211> 780
   <212> DNA
   <213> Homo sapiens
<400> 3
   ccgaattcat gggggtgcac gaatgtcctg cctggctgtg gcttctcctg tccctgctgt 60
   cgctccctct gggcctccca gtcctgggcg ccccaccacg cctcatctgt gacagccgag 120
   tcctggagag gtacctcttg gaggccaagg aggccgagaa tatcacgacg ggctgtgctg 180
   aacactgcag cttgaatgag aatatcactg tcccagacac caaagttaat ttctatgcct 240
   ggaagaggat ggaggtcggg cagcaggccg tagaagtctg gcagggcctg gccctgctgt 300
   cggaagctgt cctgcggggc caggccctgt tggtcaactc ttcccagccg tgggagcccc 360
   tgcagctgca tgtggataaa gccgtcagtg gccttcgcag cctcaccact ctgcttcggg 420
   ctctgggagc ccagaaggaa gccatctccc ctccagatgc ggcctcagct gctccactcc 480
   gaacaatcac tgctgacact ttccgcaaac tcttccgagt ctactccaat ttcctccggg 540
   gaaagctgaa gctgtacaca ggggaggcct gcaggacagg ggacagatga ccaggtgtgt 600
   ccacctgggc atatccacca cctccctcac caacattgct tgtgccacac cctcccccgc 660
   cactcctgaa ccccgtcgag ggctctcagc ctcagcgcca gcctgtccca tggacactcc 720
   agtgccagca atgacatctc aggggccaga ggaactgtcc agagagcaac tctgagatct 780
<210> 4
   <211> 193
   <212> PRT
   <213> Homo sapiens
<400> 4
   Met Gly Val His Glu Cys Pro Ala Trp Leu Trp Leu Leu Leu Ser Leu
   1 5 10 15
   Leu Ser Leu Pro Leu Gly Leu Pro Val Leu Gly Ala Pro Pro Arg Leu
   20 25 30
   Ile Cys Asp Ser Arg Val Leu Glu Arg Tyr Leu Leu Glu Ala Lys Glu
   35 40 45
   Ala Glu Asn Ile Thr Thr Gly Cys Ala Glu His Cys Ser Leu Asn Glu
   50 55 60
   Asn Ile Thr Val Pro Asp Thr Lys Val Asn Phe Tyr Ala Trp Lys Arg
   65 70 75 80
   Met Glu Val Gly Gln Gln Ala Val Glu Val Trp Gln Gly Leu Ala Leu
   85 90 95
   Leu Ser Glu Ala Val Leu Arg Gly Gln Ala Leu Leu Val Asn Ser Ser
   100 105 110
   Gln Pro Trp Glu Pro Leu Gln Leu His Val Asp Lys Ala Val Ser Gly
   115 120 125
   Leu Arg Ser Leu Thr Thr Leu Leu Arg Ala Leu Gly Ala Gln Lys Glu
   130 135 140
   Ala Ile Ser Pro Pro Asp Ala Ala Ser Ala Ala Pro Leu Arg Thr Ile
   145 150 155 160
   Thr Ala Asp Thr Phe Arg Lys Leu Phe Arg Val Tyr Ser Asn Phe Leu
   165 170 175
   Arg Gly Lys Leu Lys Leu Tyr Thr Gly Glu Ala Cys Arg Thr Gly Asp
   180 185 190
   Arg

## Description

### TECHNICAL FIELD

The present invention relates to a method for production of human erythropoietin. More specifically, the present invention relates to a process for production of human erythropoietin by means of repetitive culture of erythropoietin-producing mammalian cells following a predetermined procedure of medium exchange, and a method for production, from human erythropoietin thus obtained in the culture supernatant, of human erythropoietin which is of such high purity as may be directly used as a medical drug, without using an organic solvent and in high yield.

### BACKGROUND ART

Human erythropoietin (hEPO) is a glycoprotein which boosts the production of erythrocytes by acting on erythroblast progenitor cells to promote their differentiation into erythrocytes. By this reason, human erythropoietin has been used as an agent for the treatment of human anemia, renal anemia among others, as well as for providing a stock of autologous blood in preparation for a surgery. Artificial production of human erythropoietin is currently performed using mammalian cells which are transformed with the gene coding for it, in which culture is generally carried out in a medium containing fetal bovine serum in order to accelerate cell growth (see Non-patent Document 1). However, since the cells are cultured in a serum-containing medium there, the method cannot be free of the risk that human erythropoietin thus obtained could be contaminated with viruses and prions that may have come from the serum.

There have been reported methods in which cells are first precultured and grown in a serum-containing medium and then, after replacement of the medium with a serum-free medium, allowed to produce human erythropoietin, which is then subjected to a purification process (see Patent Documents 1 and 2). According to these methods, the risk of contamination with viruses or prions might be reduced, because serum is used in the step of preculture only, and, in the following step of human erythropoietin production, the medium has already been replaced with a serum-free medium. However, this cannot eliminate the concern about possible contamination, for just a trace amount of serum components might still have been brought into the serum-free medium used in the step of erythropoietin production. Therefore, it is thought proper to conduct the step of preculture (i.e., a step dedicated to proliferation of the cells) also, if possible, using a serum-free medium.

With this regard, there is a report describing a method for production of erythropoietin using a serum-free medium (see Patent Document 3). However, as the serum contains molecules which strongly activate cell proliferation, using no serum actually is of great disadvantage for the growth of human erythropoietin-producing cells. Especially, in the case where mass production of human erythropoietin for medical use is attempted, no-use of serum would makes designing of the process impossible or impractical, for, by using no serum, gradual reduction in the growth rate of the cells would occur with time in the long term repetitive cell culture, which is necessary for performing mass production. For that reason, there is a need for a method which provides practical long term repetitive culture of human erythropoietin-producing cells without using serum.

On the other hand, as mentioned above, there is a report of a method for production of erythropoietin using a serum-free medium (Patent Document 3). However, no description is given in the report as to a method for purification of erythropoietin produced in a serum-free medium.

As "efficient" methods to purify human erythropoietin, those employing reversed-phase chromatography have been known (Patent Documents 1 and 2). In the latter, a method is disclosed in which purification of erythropoietin, which has been produced by culture of the cells after replacement of the medium with a serum-free one, is performed using CM-sepharose chromatography and reversed-phase chromatography. Acetonitrile, however, is used there for elution of erythropoietin from the reversed-phase chromatography column. In this case, as 15.5 µg of erythropoietin was recovered out of 200 µg of it contained before the purification process was started, the yield of erythropoietin there can be calculated to be about 7.8%. And the purity of thus purified erythropoietin is no higher than 62%. Furthermore, in Patent Document 1, culture supernatant was applied to reversed-phase chromatography, in which ethanol was used for purification of erythropoietin.

As mentioned in Patent Documents 1 and 2, as far as reversed-phase chromatography is applied in the process for purification of erythropoietin, it is inevitable that an organic solvent is used. Use of an organic solvent, however, may disrupt the conformation of erythropoietin. Furthermore, considering industrial production, use of an organic solvent in the process of production makes it necessary to have an extra facility for treatment of the waste water. Therefore, it is desirable to avoid using an organic solvent either from the economical or environmental point of view.

Again, a method has been known for purification of erythropoietin from the supernatant of the cell culture performed in a serum-free medium, in which the supernatant is subjected to ultrafiltration, cation exchange chromatography, reversed phase chromatography, and gel filtration chromatography, in the order (Patent Document 4). In this process, 50∼80 % of aqueous ethanol was used to elute erythropoietin from a reversed-phase chromatography column. Since ethanol can denature proteins, there is a risk that conformation of erythropoietin may be disturbed during the process. And, although it is mentioned in the document that the yield of purified erythropoietin by the method was at least 50%, no description is given as to the level of purity of the erythropoietin obtained by the method.

A method is disclosed for purification of erythropoietin from the supernatant of a cell culture based on a serum-free medium, in which the supernatant is consecutively subjected to dye affinity chromatography, hydrophobic chromatography, hydroxyapatite chromatography, reversed phase chromatography, and anion exchange chromatography (Patent Document 5). According to this method erythropoietin was purified at higher than 99 %. However, as organic solvents such as isopropanol and acetonitrile were used in the process of purification, there was a risk that the conformation of erythropoietin might be disturbed. Furthermore, the process is not desirable for industrial scale production of erythropoietin either from the economical or environmental point of view.

A method is also known for purification of erythropoietin without using an organic solvent (Patent Document 6). In the method, the supernatant of the cell culture performed in a serum-free medium was consecutively subjected to phenylboronate chromatography, anion exchange chromatography, ultrafiltration, and gel filtration chromatography to obtain purified erythropoietin (Patent Document 6). However, the purity of erythropoietin thus recovered from the process for purification was no higher than 92 %, which is too low to allow the erythropoietin to be used as a medical drug.
[Patent Document 1] Japanese Patent Publication H4-35159
[Patent Document 2] Japanese Patent Publication H1-44317
[Patent Document 3] Japanese Patent Application Publication H5-252942
[Patent Document 4] Japanese Patent Publication H6-98019
[Patent Document 5] Japanese Patent 3061200
[Patent Document 6] Japanese Patent Application Publication 11-503726

[Non-patent Document 1] Park JH., Biotechnol. Appl. Biochem. 32, 167-172, 2000

EP0267678 discloses the production of human recombinant EPO in mouse epitheloid 0127 stably transfected cell line in serum free medium. At a density of 1.75-2.5x10⁶ cells/ml, the cell medium is changed with serum free medium. Every 48 hours 80% of the conditioned serum free medium is replaced with fresh serum free medium. The cell clone was still producing recombinant human EPO after 32 days.

US 2002/146771 describes the use of hydroxyapatite column for the separation of recombinant human EPO after a triazine dye column chromatography step.

EP1428878 discloses a method for production of human erythropoietin using a repeated batch process.

### DISCLOSURE OF INVENTION

### [The problem to be solved by the Invention]

Accordingly, it is an objective of the present invention to provide a method which enables to perform a large scale production of human erythropoietin in a medium which substantially, or entirely, is free of serum.

Another objective of the present invention is to provide a method for preparation, at high yield, of human erythropoietin having high enough a purity to allow it to be directly used as a medical drug, starting with the erythropoietin produced as above and without employing an organic solvent in the process of purification.

### [The means to Solve the Problem]

The present inventors found that, even when a serum-free medium is used for culture of human erythropoietin-producing cells, repetitive medium exchange conducted under limited and predetermined conditions makes it possible to let the human erythropoietin-producing cells continuously grow at a substantially constant growth rate over an extended period of time, without causing a decline in the growth rate of the cells. In addition, the present inventors found that, starting with the erythropoietin thus obtained in the culture supernatant, highly purified human erythropoietin can be obtained through a purification process involving a combination of dye affinity chromatography, hydroxyapatite chromatography, cation exchange chromatography, and gel filtration column chromatography. The present invention has been completed based on these findings and further examinations. Thus the present invention provides what follows below.

1. A method for production of human erythropoietin comprising the steps of:
   (a) culturing human erythropoietin-producing mammalian cells in a serum-free medium with repetitive medium exchanges, wherein each of the medium exchanges is carried out by collecting 80 to 95% of the culture when the density of the viable cells in the culture has reached 2 x 10⁶ - 4 x 10⁶ cells/ml and combining the same amount of the fresh medium with the remaining part of the culture,
   (b) preparing culture supernatant containing human erythropoietin by removing the cells contained in the culture collected in step (a)
   (c) applying the culture supernatant obtained in step (b) to dye affinity column chromatography and collecting a fraction containing human erythropoietin activity,
   (d) applying the fraction collected in step (c) to hydroxyapatite column chromatography, and collecting a fraction containing human erythropoietin activity,
   (e) applying the fraction collected in step (d) above to cation exchange chromatography, and collecting a fraction containing human erythropoietin activity, and
   (f) applying the fraction collected in step (e) above to gel filtration column chromatography, and collecting a fraction containing human erythropoietin activity, in the order,
      with the proviso that no organic solvent is used.
      (2) The method as defined in (1) above, wherein the dye in the dye affinity column chromatography is blue triazine.

### [Effect of the Invention]

The method for production of the present invention enables large scale production of human erythropoietin by serum-free process. The method, therefore, makes it possible to stably supply human erythropoietin which is substantially free of the risk of viral or prion contamination, which otherwise might be derived from the use of serum. Further, according to the method for purification of the present invention, no organic solvent is used in the process of purification, and, therefore, the risk of solvent-induced disturbance or the denaturation of human erythropoietin is eliminated. Furthermore, since an extra facility for treatment of the waste water containing organic solvent becomes no more necessary, the present invention has economical advantages and also is desirable from the environmental point of view. Still further, when serum-free medium is used, not only in the medium for production of human erythropoietin, but also in the medium for cell growth, the risk of contamination with viruses and prions, which otherwise might derived from the use of serum, is completely eliminated.

### BRIEF DESCRIPTION OF DRAWINGS

Fig. 1 illustrates vector pCI-neo into which a DNA encoding human erythropoietin is to be incorporated.
Fig. 2 illustrates vector pCI-neo(hEPO) carrying incorporated a DNA encoding human erythropoietin.
Fig. 3 is a graph showing the time-profile of the viable cell density in main culture-1.
Fig. 4 is a graph showing the time-profile of the hEPO concentration in the culture supernatant in main culture-1.
Fig. 5 is a graph showing the time-profile of the viable cell density in main culture-2.
Fig. 6 is a graph showing the time-profile of the hEPO concentration in the culture supernatant in main culture-2.
Fig. 7 is a graph showing the time-profile of the viable cell density in main culture-3.
Fig. 8 is a graph showing the time-profile of the hEPO concentration in the culture supernatant in main culture-3.

### BEST MODE FOR CARRYING OUT THE INVENTION

The human erythropoietin-producing cells used in the present invention can be generated from the cells (preferably, mammalian cells such as CHO cells derived from Chinese hamster ovary) transformed by incorporation of human erythropoietin gene using the method well known to those skilled in the art, as described in the part of examples

In the present invention, an example of a serum-free medium for performing the culture of human erythropoietin-producing cells consists of 3-700 mg/L of amino acids, 0.001-50 mg/L of vitamins, 0.3-10 g/L of monosaccharides, 0.1-10000 mg/L of inorganic salts, 0.001-0.1 mg/L of trace elements, 0.1-50 mg/L of nucleosides, 0.001-10 mg/L of fatty acids, 0.01-1 mg/L of biotin, 0.1-20 µg/L of hydrocortisone, 0.1-20 mg/L of insulin, 0.1-10 mg/L of vitamin B₁₂, 0.01-1 mg/L of putrescine, 10-500 mg/L of sodium pyruvate, and water-soluble iron compounds. The medium may be supplemented with a conventionally used pH indicator and antibiotics.

As a serum-free medium, DMEM/F12 medium (a mixture of DMEM and F12 medium) may also be used as a basal medium, which is well known to those skilled in the art. Further, as a serum-free medium, DMEM(HG)HAM modified (R5) medium, which consists of sodium hydrogen carbonate, L-glutamine, D-glucose, insulin, sodium selenite, diaminobutane, hydrocortisone, iron sulfate(II), asparagine, asparatic acid, serine, and polyvinyl alcohol, may be used. Still further, one of commercially available serum-free medium, such as CHO-S-SFM II (Invitrogen), IS CHO-V-GS (Irvine Scientific), EX-CELL302 (JRH) may also be used as a basal medium.

In the present invention, "medium exchange" is carried out, preferably, by collecting 80 to 95% of the culture, more preferably by collecting 85 to 95% (e.g., 90%), and combining the same amount of the fresh medium as the collected amount with the remaining part of the culture, when the density of the viable cells in the culture has reached at 2 x 10⁶ ∼ 4 x 10⁶ cells/mL. As a result, successive culture is started under the condition where the density of viable cells in the serum-free medium is reduced to 5 to 20 percent of the density immediately before the medium exchange (more preferably, 5 to 15%, e.g., 10%), which makes it possible to prevent the decline in the growth rate of the cells from occurring in early stages as medium is exchanged repeatedly and, thus enabling to carry out repetitive culture for an extended length of time. The temperature for the culture may be what is conventional for mammalian cell culture, e.g., 37 °C. Medium exchange is carried out, more preferably, when the density of viable cells is 2.2 x 10⁶ ∼ 4 x 10⁶ cells/mL, and still more preferably 2.5 x 10⁶ ∼ 3.8 x 10⁶ cells/mL.

Alternatively medium exchange may be carried out while adjusting the amount of the culture medium to be exchanged so that the density of viable cells immediately after each medium exchange (i.e., the density of the viable cells which was left uncollected and then diluted into the original culture volume as a result of supplementation of a fresh portion of the medium) might fall within the range of 1.5 x 10⁵ ∼ 2.5 x 10⁵ cells/mL (e.g., 2 x 10⁵ cells/mL). In this case, the culture (37 °C) following the medium exchange may be carried out either for, preferably 2-8 days, more preferably 2-6 days, and still more preferably 3-5 days, or until the density of the viable cells in the culture reaches, preferably, 2 x 10⁶ ∼4 x 10⁶ cells/mL, more preferably 2.2 x 10⁶ ∼ 4 x 10⁶ cells/mL, still more preferably 2.5 x 10⁶ ∼3.8 x 10⁶ cells/mL, and then the next medium exchange may follow.

In the repetitive culture involving medium exchanges according to the present invention, medium exchange is carried out preferably at least 4 times, more preferably at least 6 times, still more preferably at least 10 times, and particularly preferably at least 15 times. According to the method of the present invention, decline in the cell growth rate is not observed even after a repetitive culture through a number of medium exchanges.

In the present invention, all the chromatography steps for purification of hEPO are carried out in the presence of a nonionic surfactant in order to prevent nonspecific adsorption of proteins. Examples of preferred surfactants include, but are not limited to, polysorbate series surfactants, of which polysorbate 80 is particularly preferred. The concentration of a nonionic detergent is preferably 0.01 to 0.001 %(w/v), more preferably 0.005 %(w/v).

Though the process for purification of hEPO may be carried out either at room temperature or at lower temperatures, it is preferably carried out at lower temperatures, inter alia, at 1 to 10 °C.

Dye affinity chromatography, which is performed in the first step of purification, is mainly for removing of contaminants including proteases. While Blue triazine dye may preferably be used, other triazine dyes may also be properly used. A particularly preferable material for the stationary phase is Blue Sepharose 6 FF (Fast Flow) shown in the following schematic formula, which consists of Sepharose 6 Fast Flow matrix to which a dye, Cibacron™ Blue F3GA, is attached by a covalent bond.

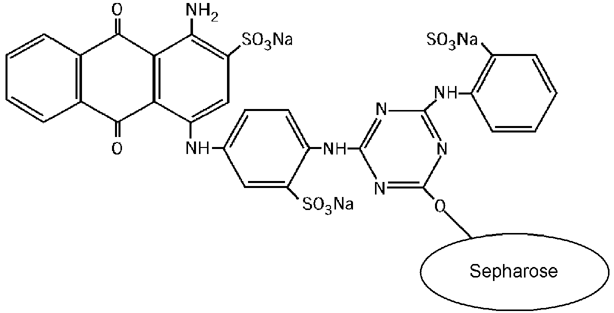

Human erythropoietin is loaded on a dye affinity chromatography column to allow it to be adsorbed by the column, which has been equilibrated around the neutral pH, and then is eluted with increasing salt concentration. Examples of the salts used for the elution include, but are not limited to, sodium chloride, whose concentration is preferably 0.2 to 1 mol/L, and more preferably 0.3 to 0.4 mol/L.

In the second step of purification, hEPO is allowed to be adsorbed by a hydroxyapatite column which has been equilibrated around the neutral pH. After washing the column, hEPO is eluted with phosphate buffer solution. The concentration of the phosphate buffer in the solution is preferably 10 to 100 mM, more preferably 20 mM. In this step, hEPO is further separated from contaminant proteins which were not removed in the first step of the purification, and thus highly purified.

After the first and the second steps of purification, a step of hydrophobic chromatography may be placed, as desired, using hydrophobic resin such as phenyl-sepharose. In that case, it is preferred that fractions containing hEPO have been adjusted in advance to pH 7.0 to 8.0 with Tris-HCl buffer and added sodium chloride at a concentration of 1.5 mol/L or more.

Cation exchange chromatography in the third step of the purification is mainly for removing the dye. Examples of cation exchange resins include, but are not limited to, preferably ion-exchange cellulose, more preferably SP-Sepharose. The fractions containing hEPO preferably are adjusted to pH5 or lower, more preferably to pH4.5 to 5.0, by a buffer solution before their application to cation exchange chromatography. Examples of buffer solutions include, but are not limited to, acetate buffer. hEPO is adsorbed by the column which has been equilibrated to pH4.5 to 5.0 with the same buffer solution, and eluted, preferably, with increasing salt concentration. Examples of salts used for this include, but are not limited to, preferably sodium chloride, whose concentration is preferably 0.05 to 0.5 mol/L, more preferably 0.1 to 0.4 mol/L, and particularly preferably about 0.3 mol/L.

Gel filtration column chromatography in the forth step of purification is mainly for removing multimeric or degraded hEPO, and it gives substantially pure hEPO.

The present invention will be described in further detail below with reference to examples.

### EXAMPLES

### [Construction of Expression Vector for Human Erythropoietin]

cDNA encoding human erythropoietin was PCR amplified from human fetal liver cDNA library (Clontech) using the following set of primers:
5'-CCGAATTCATGGGGGTGCACGAATGTCC-3' (SEQ ID NO:1), and
5'- TCAAGCTTCTTAGATCTCAGAGTTGCTCTC -3' (SEQ ID NO:2).

In the sequence set forth as SEQ ID NO: 1, nucleotides 3-8 correspond to an EcoRI site, and the nucleotides 9-28 correspond to the first 20 nucleotides of the coding region.

In the sequence set forth as SEQ ID NO: 2, nucleotides 12-17 correspond to a BglII site, and nucleotides 18-30 correspond to the nucleotides 774-762 of the cDNA.

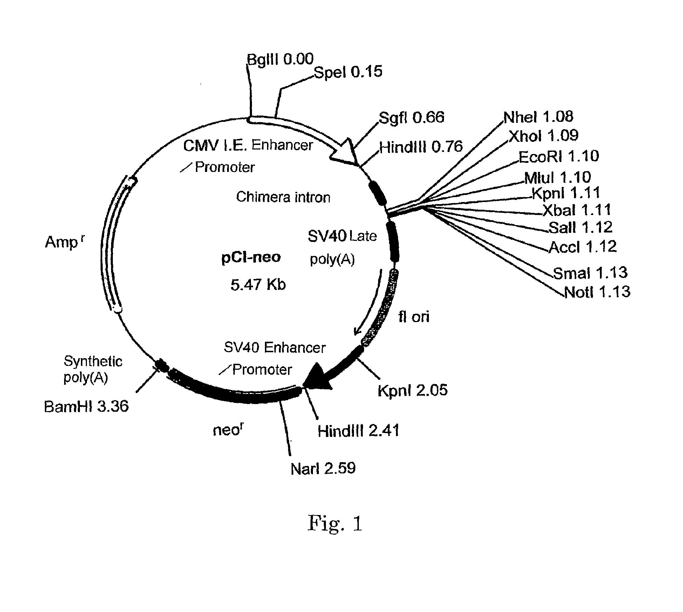

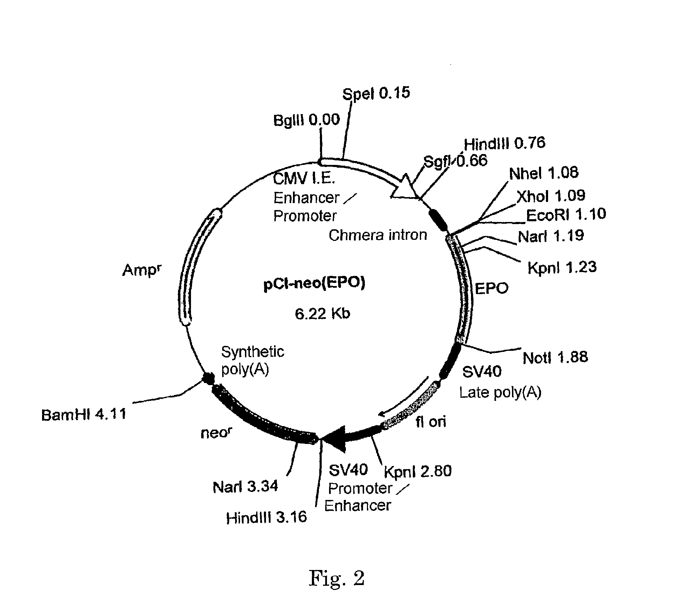

PCR reaction was cycled 30 times with denaturation step at 95 °C for 1 minute, annealing step at 60 °C for 1 minute, and elongation step at 72 °C for 2 minutes. Amplified cDNA was digested with EcoRI and HindIII, and then subcloned between the EcoRI and HindIII sites of the multicloning site of pBluescript vector (pBS: Stratagene). The resulting vector was digested with BglII, then blunt-ended by T4 DNA polymerase, and subsequently a fragment of human erythropoietin gene was excised by EcoRI digestion. The nucleotide sequences of the obtained fragment of hEPO gene and the encoded amino acid sequence are shown in SEQ ID NO:3 and NO:4, respectively. In SEQ ID NO:3, the nucleotides 9-587 correspond to the region encoding amino acids, nucleotides 3-8 correspond to EcoRI site, and nucleotides 775 to 780 correspond to BglII site. The obtained fragment of hEPO gene was ligated between EcoRI and SmaI sites of pCI-neo vector (Promega, Fig. 1) using DNA ligation kit ver.2 (TAKARA), and the resulting recombinant vector as shown in Fig. 2 was used as human erythropoietin expression vector pCI-neo(hEPO).

### [Establishment of Cells for Expression of Recombinant Human Erythropoietin]

CHO cells (CHO-K1) were purchased from RIKEN, Japan. 1 x 10⁷ CHO cells were transformed with 20 µg of the above human erythropoietin expression vector using Lipofectamine2000 (Invitrogen) in a conventional manner, then the cells were selective-cultured in the Ham-F12 medium (Invitrogen) supplemented with 10 % of fetal bovine serum (FBS) and 0.8 mg/mL of G418 (SIGMA) to obtain stable transformants which were resistant to neomycin. Then, for naturalization of the cells to a serum free medium, the cells were transferred to a commercially available serum-free EX-CELL302 medium (JRH) supplemented with 4 mM L-glutamine, 10 mg/L of hypoxanthine, 4 mg/L of thymidine, and 0.12 mg/mL of G418, and continuously cultured until the growth of cells became stable. The resulting cells were suspended in Ham-F12 medium supplemented with 10 % of FBS and 10 % of DMSO, and stored in liquid nitrogen as master cells.

### [Preculture of Cells Expressing Recombinant Human Erythropoietin]

The master cells were diluted to the cell density of 2 x 10⁵ cells/mL and incubated at 37 °C under 5 % of CO₂ in EX-CELL302 medium (JRH) supplemented with 4 mM L-glutamine, 10 mg/L of hypoxanthine, 4 mg/L of thymidine, and 0.12 mg/mL of G418 in 225 cm² culture flask. The cells were successively subcultured every 3 to 4 days, and this was repeated 3 to 6 times until the total cell number reached at least 1 x 10⁹ cells, which were required to start a 5 L-scale culture. Using the cells thus obtained, the following three different types of main culture were performed.

### [Main culture-1: 70 % medium exchange]

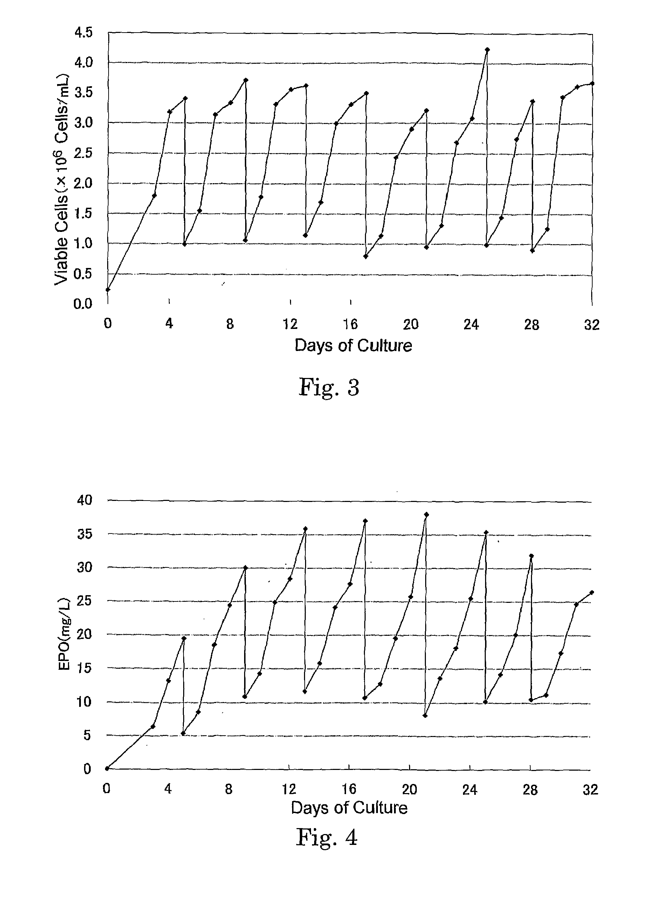

The cells obtained by the preculture were transferred to a 5-L jar fermenter (Able) so as to make the viable cell density of about 2 x 10⁵ cells/mL, and cultured at 37 °C under 5 % of CO₂ at an agitation rate of 40 rpm in the same medium as used in the preculture. Subculture of the cells was continued while replacing a 70-% part of the culture with a fresh medium once in every 3 to 6 days. The density of the viable cells was monitored with time (Fig. 3). The density of the viable cells immediately after each medium exchange was about 0.7-1.2 x10⁶ cells/mL. Small portions of the culture were sampled with time, centrifuged, and the concentration of hEPO in the obtained culture supernatant was measured by ELISA as described below (Fig. 4). The collected culture medium in each medium exchange was centrifuged to obtain the supernatant of the culture by removing the cells. The culture supernatant was frozen stored, and all of the culture mediums were thawed and combined at the end of the whole culture schedule and provided for the purification of hEPO.

During the course of culture, the maximum level of concentration of hEPO in culture supernatant was reached (about 37 mg/L) in the fifth round of subcultures. However, in the subsequent subcultures, it was found that the concentration of hEPO rapidly decreased as shown in Fig. 4, in spite that the overall viable cell density was not changed significantly. Furthermore, the levels of maximum concentration of hEPO in the subcultures were very unstable throughout the whole culture. Though the reason for this rapid decrease in the levels of maximum concentration of hEPO after the fifth round of subcultures was not clear, it may be due to the loss of the cells' ability to produce hEPO and/or decomposition of hEPO by enzymes such as proteases which were carried over through the process of medium exchange.

### [Main culture-1: 90 % medium exchange]

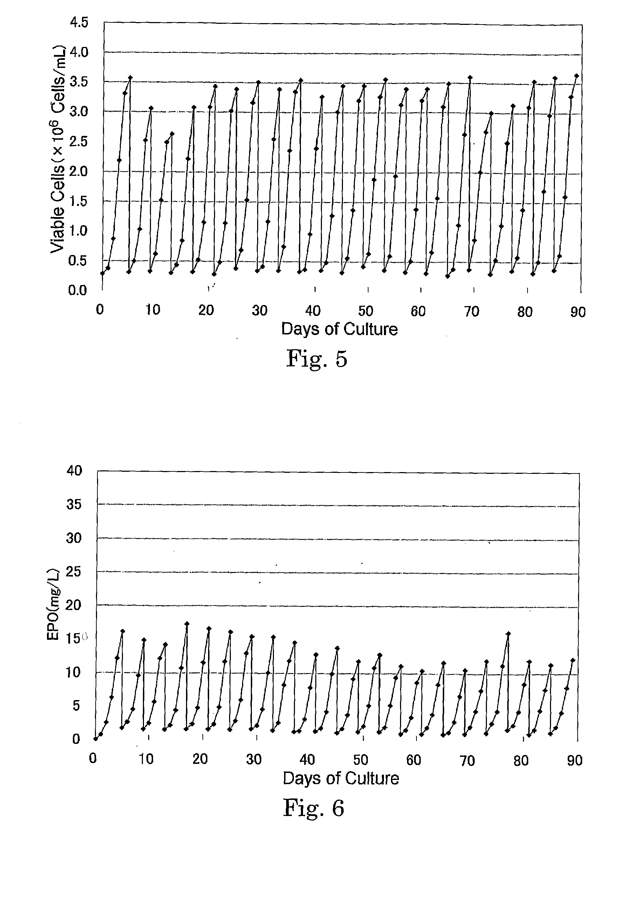

The cells obtained by the preculture were transferred to a 5-L jar fermenter (Able) so as to make the viable cell density of about 2 x 10⁵ cells/mL, and cultured at 37 °C under 5 % of CO₂ at an agitation rate of 40 rpm in the same medium as used in the preculture. Subculture of the cells was continued while replacing a 90-% part of the culture with a fresh medium once in every 3 to 6 days. The density of the viable cells was monitored with time (Fig. 5). The density of the viable cells immediately after each medium exchange was about 2.5 x 10⁵ - 4.5 x 10⁵ cells/mL. Small portions of the culture were sampled with time, centrifuged, and the concentration of hEPO in the obtained culture supernatant was measured by ELISA as described below (Fig. 6). The collected culture medium in each medium exchange was centrifuged to obtain the supernatant of the culture by removing the cells. The culture supernatant was frozen stored, and all of the culture mediums were thawed and combined at the end of the whole culture schedule and provided for the purification of hEPO.

As shown in Fig. 6, the levels of maximum concentration of hEPO in the subcultures were largely uniform even when the medium exchange was repeated twenty one times, and retained in the range of from 11 to 17 mg/L. Neither a symptom of a loss of the cells' ability to produce hEPO nor the accelerated decomposition of hEPO, such as the decrease in the concentration of hEPO, was observed at all.

### [Main culture-3: Initial number of viable cells fixed]

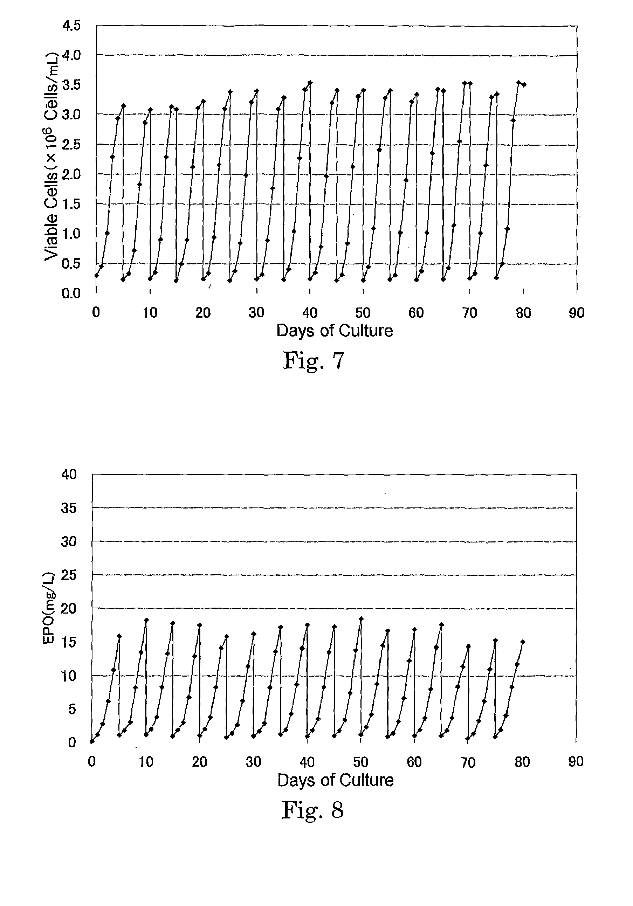

The cells obtained by the preculture were transferred to a 5-L jar fermenter (Able) so as to make the viable cell density of about 2 x 10⁵ cells/mL, and cultured at 37 °C under 5 % of CO₂ at an agitation rate of 40 rpm in the same medium as used in the preculture. Subculture of the cells was continued while repeating medium exchange once in every 3 to 6 days. The density of the viable cells was monitored with time (Fig. 7). Based on the density of viable cells measured at the time of each medium exchange, the amount of the culture medium to be exchanged was adjusted so that the density of the viable cells immediately after each medium exchange (i.e., the density of the viable cells which was left uncollected and then diluted into the original culture volume as a result of supplementation of a fresh portion of the medium) might come around 2.5 x 10⁵ cells/mL. Small portions of the culture were sampled with time, centrifuged, and the concentration of hEPO in the obtained culture supernatant was measured by ELISA as described below (Fig. 8). The collected culture medium in each medium exchange was centrifuged to obtain the supernatant of the culture by removing the cells. The culture supernatant was frozen stored, and all of the culture mediums were thawed and combined at the end of the whole culture schedule and provided for the purification of hEPO.

As shown in Fig. 8, the levels of maximum concentration of hEPO in the subcultures were largely uniform even when the medium exchange was repeated fifteen times, and retained in the range of from 15 to 19 mg/L. Neither a symptom of a loss of the cells' ability to produce hEPO nor the accelerated decomposition of hEPO, such as the decrease in the concentration of hEPO, was observed at all.

### [Method for purification of human erythropoietin]

After the pH of the culture supernatant was adjusted to about 6.8, its conductivity was adjusted to not more than 13 mS/cm by the addition of distilled water containing 0.005 %(w/v) of polysorbate 80. About 160 mL of the culture supernatant was applied to Blue-Sepharose FF (16 mm x 75 mm; 15 mL, Amersham) that had been pre-equilibrated in advance with 0.05 M Tris buffer (pH 7.5) containing 0.005%(w/v) of polysorbate 80, at a flow rate of 120 cm/hour, thereby allowing human erythropoietin to be adsorbed by the resin. Then, the column was washed with 75 mL of the above buffer, and, subsequently, human erythropoietin was collected by elution with 0.05 M Tris buffer (pH 7.5) containing 0.005 %(w/v) of polysorbate 80 and 0.4 M sodium chloride, at the same flow rate.

Then calcium chloride was added to the eluted fractions from the above Blue-Sepharose FF column chromatography in such a manner that its final concentration came to 2 mM, and, subsequently, the fraction was applied to a hydroxyapatite column (HA ULTROGEL: 26 mm x 56 mm; 30 mL, BioSepra) that had been pre-equilibrated with 0.05 M Tris buffer (pH 7.5) containing 1 M sodium chloride and 2 mM calcium chlorite, at a flow rate of 45 mL/hour, thereby allowing human erythropoietin to be adsorbed by the resin. The column was subsequently washed at the same flow rate with 150 mL of the above buffer, and then human erythropoietin was collected by elution with 20 mM phosphate buffer solution (pH 7.5) containing 0.005 %(w/v) of polysorbate 80.

Then, 1/10 volume of 0.05 M acetate buffer solution (pH4.5) was added to the above eluted fraction from HA ULTROGEL column chromatography to adjust the pH to not higher than 5, then distilled water containing 0.005 %(w/v) of polysorbate 80 was added further to adjust the conductivity of the fraction not more than 9 mS/cm. The diluted fraction was applied to a cation-exchange resin SP-Sepharose FF column (16 mm x 40 mm; 8 mL, Amersham) that had been pre-equilibrated with 0.05 M acetate buffer solution (pH5.0) containing 0.005 %(w/v) of polysorbate 80, at a flow rate of 40 mL/hour, thereby allowing human erythropoietin to be adsorbed by the resin. The column was subsequently washed at the same flow rate with 0.05 M acetate buffer solution (pH5.0) containing 0.005% (w/v) of polysorbate 80 and 0.05M sodium chloride, and then human erythropoietin was collected by elution with 0.05 M acetate buffer solution (pH5.0) containing 0.005%(w/v) of polysorbate 80 and 0.3M sodium chloride.

Then, the eluted fraction from the above SP-Sepharose FF column chromatography was concentrated by membrane ultrafiltration (Exclusion limit: 8000) to 1 mL of volume. Then the concentrated fraction was applied to a Superdex 200pgXK16/60 column (16 mm x 600 mm; 120 mL, Amersham) that had been pre-equilibrated with 0.05M Tris buffer containing 0.005%(w/v) polysorbate 80 and 0.2M sodium chloride, and human erythropoietin was separated, and collected by gel-filtration at 30 mL/hr.

### [Method for in vivo determination of hEPO based on biological activity to promote reticulocyte production]

As test animals, B6D2F1(BDF1) strain mice (eight-week old, male (Charles River) were used. Test samples of human erythropoietin were subcutaneously administered to the animals at the back. On the 4th days after the administration, 200 µL of blood was collected from the postcaval vein of the test animals and the number of erythrocytes (RBC, x 10⁴/µL), the number of blood reticulocytes (RET, x 10⁴/µL), and blood reticulocyte rate (RET%, %) were measured within 30 minutes on an automatic erythrocyte counter R-500 (Sysmex). The in vivo biological activity (U/mL) was calculated by comparison to that of a standard human erythropoietin (European Pharmacopoeia).

### [Purity test of human erythropoietin using SE-HPLC]

Test samples of human erythropoietin were diluted five times by a mobile phase solution containing 5 mM potassium dihydrogen phosphate, 8.1 mM sodium dihydrogen phosphate, and 0.4M sodium chloride (pH7.4). The diluted samples were applied to TSK-gel G3000SW (7.5 mm x 600 mm, TOSOH) using a HPLC system LC-10A (SHIMAZU), gel-filtrated at a flow rate of 0.5 mL/minute at room temperature, and the absorbance at 214 nm was measured.

### [Method for determination of hEPO by ELISA method]

Rabbit anti-hEPO antibody solution was prepared by a conventional method from the blood of a rabbit immunized with the hEPO prepared by the present inventors. One hundred µL of rabbit anti-hEPO antibody solution was added to each well of 96-well plate and stored at 4 °C for 1 hour for fixation. After discarding the solution, 100 µL of 1 % BSA/TBS-T solution (0.005 M Tris, 1% of BSA, 0.138 M sodium chloride, and 0.0027 M potassium chloride, pH8.0) containing 0.075% of Tween20 was added to the each well and stored at 4 °C for 1 hour for blocking. After discarding the solution, each well was washed with 100 µl of TBS-T solution containing 0.075% of Tween20 three times, and 100 µL of the sample solutions, after diluted to measurable concentrations, were added to the wells and stored at 37 °C for 1 hour. Simultaneously, to some wells were added standard solutions which had been prepared at concentrations of 1-16 ng/mL by diluting the hEPO prepared and determined, by Lowry method, by the present inventors. After discarding the solution, each well was washed as described above, and 100 µL of HRP-labeled mouse anti-hEPO monoclonal antibody (R&D) was added as the secondary antibody to each well and stored at 37 °C for 1 hour. After washing each well as described above, HRP substrate (Promega) was added to each well and stored at 37 degrees for 15 minutes, and the reaction then was terminated by addition of HCl. Absorbance at 450 nm was measured by a microwell plate reader, and the concentrations of hEPO in the samples were calculated by comparison to the absorbance with the standard solutions.

### [Purity test of human erythropoietin using reversed-phase HPLC]

Solution A consisting of 0.06% trifluoroacetic acid (TFA) and solution B consisting of 0.06% TFA/80% acetonitrile were provided. Test samples of human erythropoietin were applied to a butyl-silylated silica gel (Vydac214ATP54™, Vydac) column and eluted at a flow rate of 1.0 mL/minute at 25 °C, with a concentration gradient employing solution B at a proportion of 50-75 %, using a HPLC system LC-10A (SHIMAZU).

### [Results of analysis of human erythropoietin]

As a result of in vivo determination, the human erythropoietin obtained by the above method was found to have the specific activity of not less than 1.5 x 10⁵ U/mg, and it was revealed that about 45 % of the erythropoietin which had been contained in the culture was recovered. It was also found that the purified human erythropoietin obtained by the above method of the present invention was almost of 100 % purity, as it gave a single peak in either of the purity tests, i.e., by gel-filtration HPLC or reversed-phase HPLC.

### INDUSTRIAL APPLICABILITY

The present invention enables large scale production of human erythropoietin by an entirely serum-free process. The method, therefore, makes it possible to stably supply human erythropoietin, which is substantially free of the risk of viral or prion contamination. Further, the present invention can eliminates the risk of solvent-induced disturbance or the denaturation of human erythropoietin, which could otherwise occur during the process of its production. Furthermore, the present invention has economical advantages and also is desirable from the environmental point of view. Still further, when combined with the use of a serum-free medium in the culture dedicated to cell growth in preparation for starting the culture for hEPO production and collection, the present invention completely eliminates the risk of contamination with viruses and prions.

## Claims

1. A method for production of human erythropoietin comprising the steps of:
(a) culturing human erythropoietin-producing mammalian cells in a serum-free medium with repetitive medium exchanges, wherein each of the medium exchanges is carried out by collecting 80 to 95% of the culture when the density of the viable cells in the culture has reached 2 x 10⁶ - 4 x 10⁶ cells/ml and combining the same amount of the fresh medium with the remaining part of the culture,
(b) preparing culture supernatant containing human erythropoietin by removing the cells contained in the culture collected in step (a)
(c) applying the culture supernatant obtained in step (b) to dye affinity column chromatography and collecting a fraction containing human erythropoietin activity,
(d) applying the fraction collected in step (c) to hydroxyapatite column chromatography, and collecting a fraction containing human erythropoietin activity,
(e) applying the fraction collected in step (d) above to cation exchange chromatography, and collecting a fraction containing human erythropoietin activity, and
(f) applying the fraction collected in step (e) above to gel filtration column chromatography, and collecting a fraction containing human erythropoietin activity, in the order,
with the proviso that no organic solvent is used.

2. The method of claim 1, wherein the dye in the dye affinity column chromatography is blue triazine.

## Patentansprüche

1. Verfahren zur Herstellung von menschlichem Erythropoetin, die folgenden Schritte umfassend:
(a) Anzüchten von Säugetierzellen, die menschliches Erythropoetin produzieren, in einem serumfreien Medium mit wiederholtem Medienaustausch, wobei jeder einzelne Medienaustausch derart durchgeführt wird, dass 80 bis 95 % des Kulturansatzes entnommen werden, wenn die Dichte an lebensfähigen Zellen in dem Kulturansatz 2 x 10⁶ bis 4 x 10⁶ Zellen/ml erreicht hat, und dass eine ebenso große Menge des frischen Mediums mit dem verbleibenden Teil des Kulturansatzes vereint wird,
(b) Gewinnen des Kulturansatz-Überstandes, der menschliches Erythropoetin enthält, indem die Zellen entfernt werden, welche in dem Kulturansatz enthalten sind, der in Schritt (a) entnommen wurde.
(c) Aufbringen des Kulturansatz-Überstandes, der in Schritt (b) erhalten wurde, auf eine Chromatographieanlage mit Farbstoffaffinitätssäule und Auffangen einer Fraktion, die eine Aktivität bezüglich menschlichen Erythropoetins enthält,
(d) Aufbringen der Fraktion, die in Schritt (c) aufgefangen wurde, auf eine Chromatographieanlage mit Hydroxyapatitsäule und Auffangen einer Fraktion, die eine Aktivität bezüglich menschlichen Erythropoetins enthält,
(e) Aufbringen der Fraktion, die in Schritt (d) aufgefangen wurde, auf eine kationenaustauschbasierte Chromatographieanlage und Auffangen einer Fraktion, die eine Aktivität bezüglich menschlichen Erythropoetins enthält, und
(f) Aufbringen der Fraktion, die in Schritt (e) aufgefangen wurde, auf eine Chromatographieanlage mit Gelfiltrationssäule und Auffangen einer Fraktion, die eine Aktivität bezüglich menschlichen Erythropoetins enthält, und zwar in dieser Reihenfolge,
mit der Maßgabe, dass kein organisches Lösungsmittel verwendet wird.

2. Verfahren nach Anspruch 1, wobei es sich bei dem Farbstoff in der Chromatographieanlage mit Farbstoffaffinitätssäule um blaufarbenes Triazin handelt.

## Revendications

1. Procédé de production d'érythropoïétine humaine comprenant les étapes de :
(a) culture de cellules mammifères produisant l'érythropoïétine humaine dans un milieu sans sérum avec des échanges de milieu répétitifs, où chacun des échanges de milieu est réalisé en collectant 80 à 95 % de la culture lorsque la densité de cellules viables dans la culture a atteint 2 x 10⁶ à 4 x 10⁶ cellules/ml et en combinant la même quantité de milieu frais avec la partie restante de la culture,
(b) préparation du surnageant de culture contenant l'érythropoïétine humaine en enlevant les cellules contenues dans la culture collectée dans l'étape (a),
(c) application du surnageant de culture obtenu dans l'étape (b) à une chromatographie sur colonne d'affinité de colorant et collecte d'une fraction contenant l'activité érythropoïétine humaine,
(d) application de la fraction collectée dans l'étape (c) à une chromatographie sur colonne d'hydroxyapatite et collecte d'une fraction contenant l'activité érythropoïétine humaine,
(e) application de la fraction collectée dans l'étape (d) ci-dessus à une chromatographie d'échange de cations, et collecte d'une fraction contenant l'érythropoïétine humaine, et
(f) application de la fraction collectée dans l'étape (e) ci-dessus à une chromatographie sur colonne de filtration sur gel et collecte d'une fraction contenant l'activité érythropoïétine humaine, dans l'ordre, à la condition qu'aucun solvant organique ne soit utilisé.

2. Procédé selon la revendication 1, dans lequel le colorant dans la chromatographie sur colonne d'affinité de colorant est le bleu de triazine.
